# EUROPEAN PATENT APPLICATION

(11) **EP 1 702 622 A1**
(43) Date of publication of application: **20.09.2006**
(21) Application number: 05006000.3
(22) Date of filing: 18.03.2005
(51) Int. Cl.: A61K 38/17, A61P 37/00

(54) **Soluble BTNL2 protein useful to inhibit inflammatory disorders**

(71) Applicant: CONARIS research institute AG, 24118 Kiel (DE)
(72) Inventor: Rosenstiel, Philip, 24116 Kiel (DE); Seegert, Dirk, 24161 Altenholz (DE); Schreiber, Stefan, 24105 Kiel (DE)
(74) Representative: Schüssler, Andrea

(57) **Abstract**

Described is a polypeptide-dimer comprising at least one soluble BTNL2 molecule, preferably two soluble BTNL2 molecules. In addition, a pharmaceutical composition is described containing said molecule and various medical uses of this molecule to inhibit T-cell mediated inflammatory disorders.

## Description

The present invention relates to a polypeptide-dimer comprising at least one soluble BTNL2 molecule, preferably two soluble BTNL2 molecules. The invention also relates to a pharmaceutical composition containing said molecule and various medical uses of this molecule to inhibit T-cell mediated inflammatory disorders.

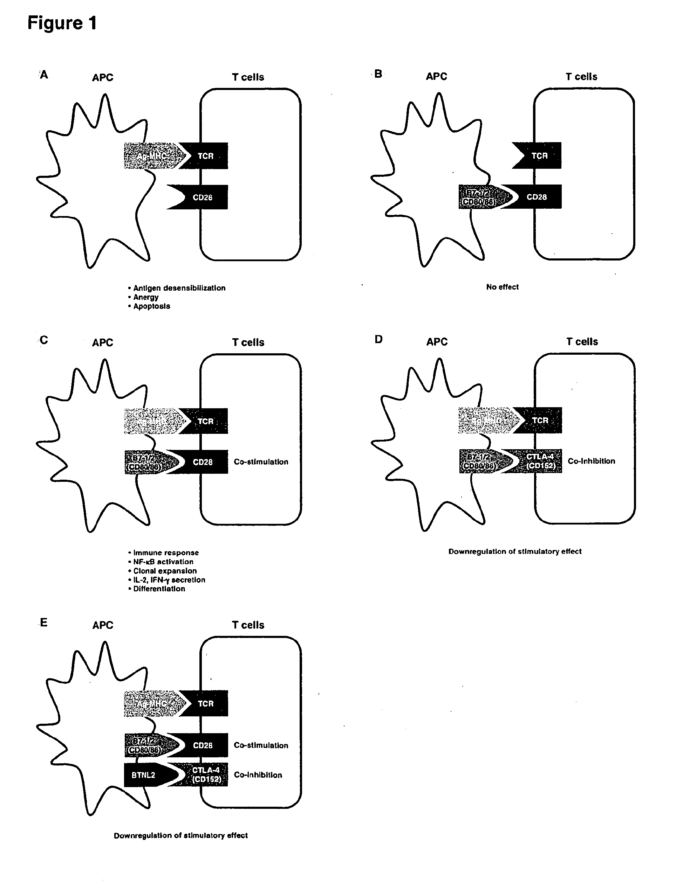

Co-stimulation between T cells and antigen presenting cells (APC) is necessary for effective immune responses. Primary signals delivered through the interaction of T cell receptor (TCR) and major histocompatibility complexes (MHC) can be modulated by co-signalling via certain cell-surface glycoproteins, e.g. CD28/B7 molecules (CD80 and CD86), CD40/CD40 ligand (CD40L, CD154), and LFA-1 (CD18)/ICAM-1 (CD54). Depending on their function, these molecules are further divided into co-stimulators and co-inhibitors of T cell activity. One of the best characterized co-signalling systems is represented by B7-1 and B7-2 on APC which deliver the signal through CD28 and cytotoxic T-lymphocyte antigen 4 (CTLA-4) on T cells. Whereas T cell responses are augmented by CD28, they are inhibited by CTLA-4 (Figure 1) (Bluestone, Clin Transplant, 1996, 10:104; Czitrom, Clin Orthop, 1996, 11; Harris, et al., Immunol Cell Biol, 1999, 77:304). Deletion of the IgC domain in the CD80 gene had a substantial pro-inflammatory effect in a mouse plasmid vaccination model, suggesting that the constant-like domains are crucial for immune regulation during co-stimulation.

The specific expression of these molecules at certain times positively and negatively controls priming, growth, differentiation and functional maturation of a T cell response. CD28 is constitutively expressed on T cells, whereas CTLA-4 is only found at low concentrations on naive T cells, but is rapidly induced after T-cell activation (Chambers, et al., Annu Rev Immunol, 2001, 19:565). Beside B7-1 and B7-2, the B7 family comprises further new members such as ICOS ligand, PD-L1 (B7-H1), PD-L2 (B7-DC), B7-H3, and B7-H4 (B7x/B7-S1) (Chen, Nat Rev Immunol, 2004, 4:336).

It has become obvious in the past that co-stimulation plays a key role in the activation of T cells and T cell mediated immune responses. The dysregulation of these highly sensitive co-stimulatory systems often results in the development of autoimmune diseases due to impaired T cell activation. A significant up-regulation of B7-2 and a simultaneous downregulation of ICAM-1 (CD54) was observed in patients with insulin-dependent diabetes mellitus (Spatz, et al., Cell Immunol, 2003, 221:15).

Increased levels of B7-1 and B7-2 have been observed on intestinal macrophages derived from either Crohn's disease or ulcerative colitis patients (Rogler, et al., Eur J Gastroenterol Hepatol, 1999, 11:1105)

Single nucleotide polymorphisms (SNP) within the CTLA-4 gene have been demonstrated to influence either the expression or the inhibitory function of the protein and to be associated with different autoimmune diseases (Ligers, et al., Genes Immun, 2001, 2:145). A SNP in exon 1 of the CTLA-4 gene was shown to be associated with the development of multiple sclerosis (MS) (Ligers, et al., J Neuroimmunol, 1999, 97:182).

Variations in CTLA-4 have also been identified as a severe risk factor for other autoimmune disorders such as type I diabetes, autoimmune hypothyroidism and Grave's disease (Ueda, et al., Nature, 2003, 423:506).

Several animal studies have shown that the disruption of CD28/B7 co-stimulation could reduce the severity of the pathology, and in some cases, could completely prevent a disease. For instance, blockade of CD28/B7 by anti-B7 antibodies or by CTLA-4 fusion proteins reduced disease severity in mouse models of multiple sclerosis (Karandikar et al., J Neuroimmunol, 1998, 89:10; Anderson, et al., Curr Opin Immunol, 1999, 11:677), myocarditis (Bachmaier, et al., J Immunol, 1996, 157:1752), arthritis (Tada, et al., J Immunol, 1999, 162:203), thyroiditis (Peterson, et al., J Immunol, 1999, 162:1859), myasthenia gravis (Shi, et al., Eur J Immunol, 1998, 28:3587), or lupus erythematosus (Finck, et al., Science, 1994, 265:1225; Kinoshita, et al., J Immunol, 2000, 164:6046).

In transplantation it has long been assumed that a profound induction of tolerance of the graft would prolong graft survival. Treatment with CTLA-4Ig at the time of implantation induced long-term acceptance of human islets in mice (Lenschow, et al., Science, 1992, 257:789) and cardiac allografts in rats (Turka, et al., Proc Natl Acad Sci U S A, 1992, 89:11102). Blockade of CD40/CD40L or CD28/B7 during transplantation induced a decrease of proliferation of alloreactive T cells and an increase of apoptosis (Li, et al., Nat Med, 1999, 5:1298; Zheng, et al., Transplant Proc, 1999, 31:627; Iwakoshi, et al., J Immunol, 2000, 164:512).

In a clinical study, CTLA-4Ig was used to treat 43 patients with psoriasis vulgaris. After 4 infusions approximately 50% of the patients achieved a sustained improvement in clinical disease activity in a dose-dependent manner (Abrams, et al., J Clin Invest, 1999, 103:1243). In another example graft-vs-host disease (GVHD) was reduced in bone marrow allograft transplantation. For this purpose alloantigen-specific anergy was induced by co-culturing bone marrow from the donor with peripheral lymphocytes from the recipient for 36 hours in the presence of CTLA-4Ig to block CD28/B7 (Guinan, et al., N Engl J Med, 1999, 340:1704).

A study in 339 patients with rheumatoid arthritis (RA) demonstrated, that a combined therapy with methotrexate and either 2 (n=105) or 10 mg (n=115) CTLA-4Ig per kilogram resulted in a significant reduction of the ACR arthritis index (Kremer, et al., N Engl J Med, 2003, 349:1907). Significant higher rates of ACR50 and ACR70 responses were seen in both verum groups as compared to the placebo controls (n=119). This study shows that CTLA-4Ig is a promising new drug for the medication of RA.

The immunopathogenesis of demyelination in multiple sclerosis involves an autoantibody response to the immunoglobulin superfamily member myelin oligodendrocyte glycoprotein (MOG). Crystal structure analysis has demonstrated that the extracelular domain of MOG adopts an IgV like fold that harbours a cavity which is similar to the one used by the co-stimulatory molecule B7-2 to bind its ligand CTLA4 (Breithaupt, et al., Proc Natl Acad Sci U S A, 2003, 100:9446).

Sarcoidosis is a systemic inflammatory disease of unknown etiology, characterized by T-lymphocyte and mononuclear phagocyte (MNP) infiltration leading to granuloma formation. The hallmark of the disease is the presence of noncaseating granulomas in the affected organs (Sharma, Dis Mon, 1990, 36:469; Kanathur, et al., South Med J, 2000, 93:631). These granulomas can occur with varying rates in any organ system, but are most commonly found in the lung and lymph nodes. A range of 30-60% of reported cases of sarcoidosis are asymptomatic and are discovered during routine health screening chest radiographs. For patients, the most common problems include respiratory symptoms such as dry cough, dyspnea or chest pain and systemic symptoms e.g. fever, night-sweats, fatigue or malaise.

Clinical presentation of the disorder varies widely, with two main phenotypes: acute and chronic sarcoidosis. Acute sarcoidosis resolves within two to three years, often disappearing spontaneously and leaving no residual effects. A subset of acute patients exhibit Löfgren's syndrome, which is chacterised by a combination of fever, bilateral hilar lymphadenopathy, erythema nodosum and arthritis, often at the ankle joints (Johard, et al., Sarcoidosis, 1993, 10:125; Oshima, et al., Intern Med, 2003, 42:534). Approximately a quarter of sarcoidosis patients have permanent lung damage due to scarring, and some may suffer from chronic sarcoidosis. In about five percent of the cases, organ transplantation (such as lungs, heart and liver) is required to avoid death.

Sarcoidosis demonstrates remarkable overlap with other chronic inflammatory disorders. Blau syndrome and Crohn's disease are complex disorders that show overlap with sarcoidosis in the spectrum of involved organs and their immunopathophysiology. Enhanced immune response to a yet unknown target with monocyte and T-cell activation and granuloma formation in affected tissues are characteristic features of both sarcoidosis and Crohn's disorders. Although the primary sites of inflammation differ (lung in sarcoidosis and intestine in Crohn's disease), affection of skin, joints and other organs is common in both disorders. Coincidence of sarcoidosis and Crohn's disease in families or even in one individual is more common than expected by chance alone.

In order to localize sarcoidosis susceptibility genes, several linkage studies and many association studies have been performed in different populations. A linkage study of seven polymorphisms spanning the MHC region in 55 German sarcoidosis families gave an estimated maximum NPL score of >2.5 for the entire MHC region, with a maximum value of 3.2 at marker locus D6S1666. Later, a detailed genome-wide linkage study of 63 German sarcoidosis families with 225 microsatellite markers identified seven peaks of linkage evidence located on six chromosomes. The most prominent peak was still located on chromosome 6 at the microsatellite marker D6S1666 with an NPL score of 2.99 (p=0.0001). A systematic three-stage single nucleotide polymorphism (SNP) scan of 16.4 Mb on chromosome 6p21 was performed in up to 947 independent cases of familial and sporadic sarcoidosis. Using TDT and case-control analyses, a 15 kb segment located at the 3'-end of the BTNL2 gene could be identified as being strongly associated with sarcoidosis. The major disease-associated variant, rs2076530, represents a risk factor that is entirely independent of the previously reported association between sarcoidosis and alleles of the DRB1 gene, located within ~200kb of BTNL2. The risk allele A of rs2076530 leads to alternative splicing of the BTNL2 transcript, which introduces a premature stop. The resulting truncated protein lacks the C-terminal IgC domain and transmembrane helix, thereby disturbing the putative coinhibitory function of this molecule. However, so far the exact biological function of BTNL2 and, thus, its possible involvement in the progression of further (inflammatory) diseases was totally unknown.

Thus, the technical problem underlying the present invention was to determine whether T cell activation by co-stimulation via CD3/CD28 could be inhibited by treatment with recombinant BTNL2 molecules.

The solution to said technical problem is achieved by providing the embodiments characterized in the claims. The present invention is based on the observation that BTNL2 inhibits CD3/CD28-mediated NF-KB activation in T-cells. It is hypothesized, that the loss of BTNL2 function by the described mutation interferes with BTNL2 dependent T cell control and augments T cell mediated immune responses. Moreover, this dysregulated T cell activity could lead to the development of chronic inflammatory disorders.

Based on homologies of amino acids and domain structures, BTNL2 is very similar to the B7-1 protein (Valentonyte et al., Nature Gentecics, 27 February 2005; doi:10.1038/ng1519). BTNL2 also shows similarity to mouse NG9 and NG10 genes and is homolog to the Zipper proteins, bg2, MOG, and B-G which, phylogenetically appear to be members of the extended B7 family.

Thus, in a first embodiment, the present invention relates to a polypeptide-dimer comprising at least one soluble BTNL2 molecule, preferably two soluble BTNL2 molecules.

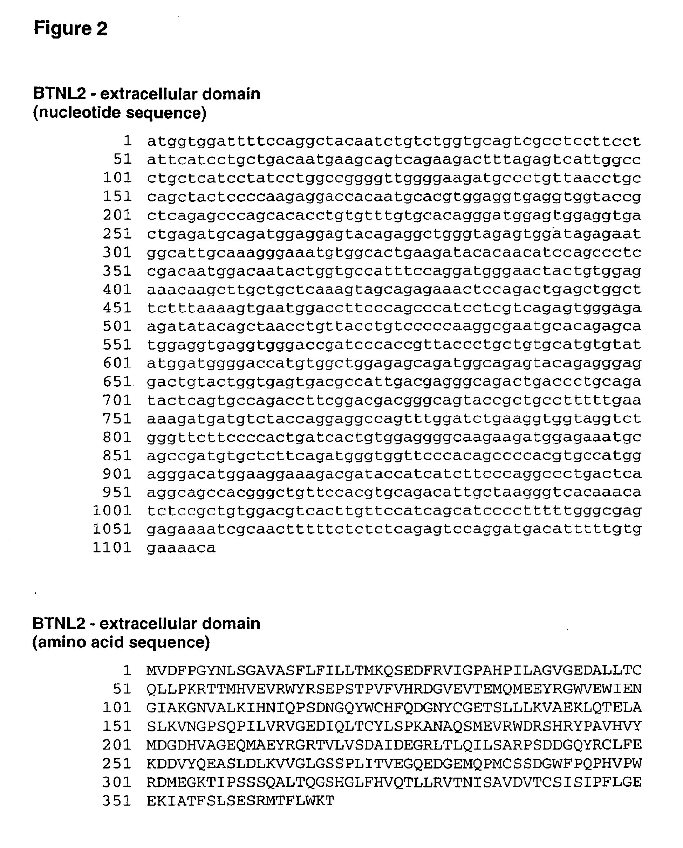

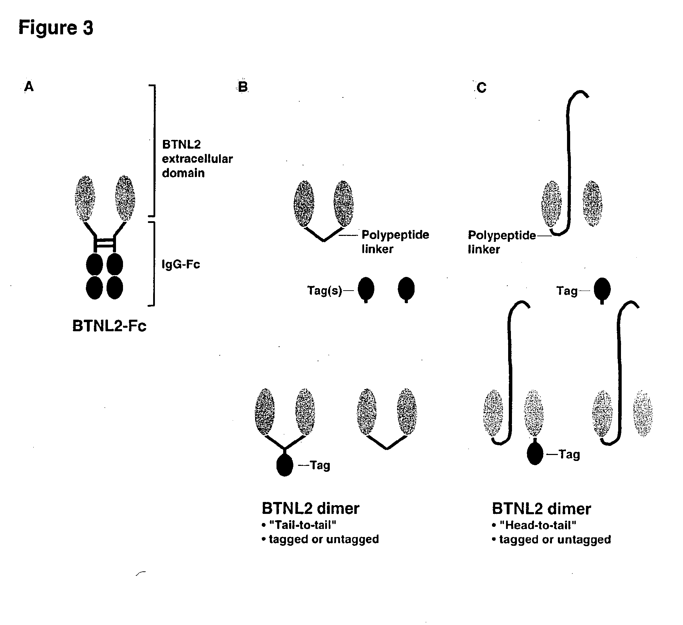

The term "polypeptide-dimer" as used herein also comprises fusion proteins containing the monomers linked via a normal peptide bond ("head-to-tail") to form concatamers or "tail-to-tail" (Figure 3B). Polypeptide-dimers of the present invention may be engineered using known methods. The soluble domains of BTNL2 utilized may consist of the entire extracellular domain of BTNL2 or they consist of mutants or fragments thereof that substantially maintain the biological activity of the entire extracellular domain. The nucleotide and corresponding amino acid sequences of the extracellular part of BTNL2 are shown in Figure 2. The extracellular domain of BTNL2 is from aa 1 (including the signal peptide) to aa 369.

In a preferred embodiment of the polypeptide-dimer at least one of the two soluble BTNL2 molecules is the entire extracellular domain of BTNL2.

The monomers of the dimer may be directly linked, i.e., the C-terminus of one polypeptide chain is linked to the N-terminus (or C-terminus) of the other through a simple covalent bond, or they may employ a flexible linker domain, such as the hinge region of human IgG, or a polypeptide linker consisting of small amino acids such as glycine, serine, threonine or alanine at various lengths and combinations. Additionally, the polypeptide-dimer of the invention may be tagged by, e.g., His₆, to allow rapid and simple purification by metal-chelate chromatography and/or by epitopes to which antibodies are available to allow detection, e.g., by western blot, immunoprecipitation etc.

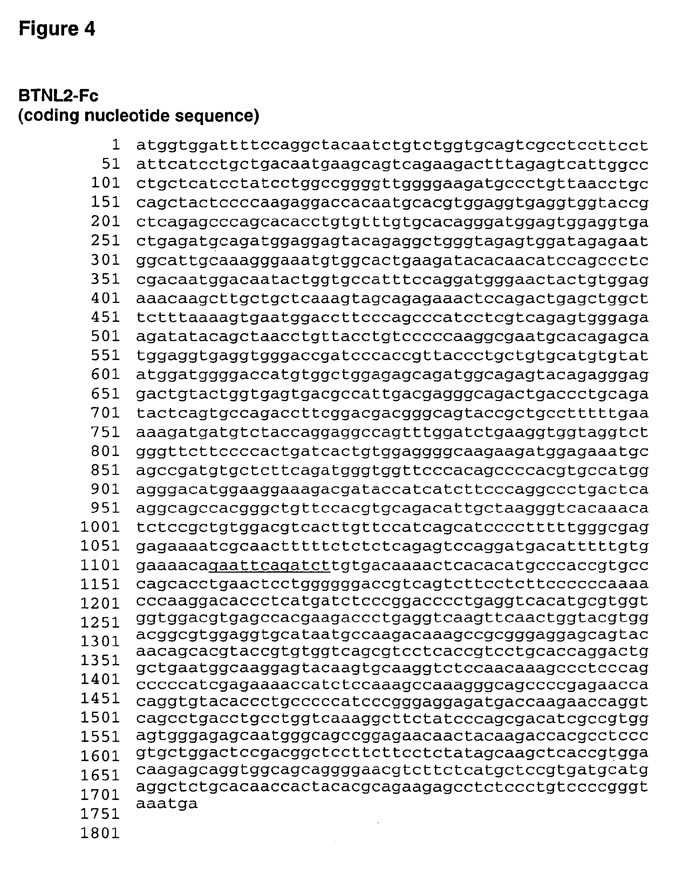

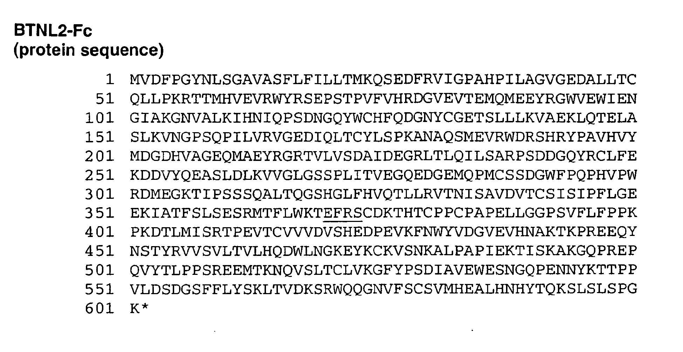

In a further preferred embodiment, in the polypeptide-dimer of the invention the two BTNL2 molecules are fused to the Fc domains of an IgG protein (see Aruffo et al., Cell 67(1) (1991), 35-44). In this case, heterodimeric molecules carrying the Fc domain are preferably expressed as disulphide-linked dimers (containing one or more disulphide bridges; Figure 3A). The amino acid sequence is shown in Figure 4.

In addition, the present invention also relates to engineered, mutated versions of soluble BTNL2 with altered properties, e.g., as regards binding.

The present invention also provides a polynucleotide encoding a dimer of the invention or a monomer of said dimer.

The polynucleotides of the invention can be both DNA and RNA molecules. Suitable polynucleotides are, for example, genomic or cDNA molecules. It is understood that all nucleic acid molecules encoding all or a portion of the soluble part of BTNL2 are also included, as long as they encode a polypeptide with biological activity. The polynucleotides of the invention can be isolated from natural sources or can be synthesized according to known methods.

The present invention also provides polynucleotides encoding a polypeptide dimmer or monomer the amino acid sequence of which shows at least 70%, preferably at least 80%, 85%, 90%, 95% or, in the most preferred embodiment, 98% identity to the amino acid sequence of the BTNL2 domains of Figure 2. Such amino acid sequences are characterized by deletion, substitution and/or insertion of amino acid residue(s) compared to the amino acid sequence shown in Figure 2 or are the result of recombination. They can be naturally occurring variations, for example sequences from other organisms, or mutations that can either occur naturally or that have been introduced by specific mutagenesis. They can also be isolated, e.g., from genomic or cDNA libraries that were produced from cells or tissues. In order to identify and isolate such polynucleotides of the invention, parts of these molecules or the reverse complements of these molecules can be used, for example by means of hybridization. As a hybridization probe nucleic acid molecules can be used, for example, that have exactly or basically the nucleotide sequence as depicted in Figure **2,** respectively, or parts of these sequences. The fragments used as hybridization probe can be synthetic fragments that were produced by means of conventional synthetic methods and the sequence of which basically corresponds to the sequence of a polynucleotide of the invention.

Generally, by means of conventional molecular biological processes it is possible to introduce different mutations into the polynucleotides of the invention. As a result, soluble BTNL2 polypeptides with possibly modified biological properties are synthesized. One possibility is the production of deletion mutants in which nucleic acid molecules are produced by continuous deletions from the 5'- or 3'-terminus of the coding DNA sequence and that lead to the synthesis of polypeptides that are shortened accordingly. Another possibility is the introduction of a single-point mutation at positions where a modification of the amino acid sequence influences, e.g., the binding properties.

For the manipulation in prokaryotic cells by means of genetic engineering the polynucleotides of the invention or parts of these molecules can be introduced into plasmids allowing a mutagenesis or a modification of a sequence by recombination of DNA sequences. By means of conventional methods bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The invention furthermore relates to vectors containing a polynucleotide of the invention. Preferably, they are plasmids, cosmids, viruses, bacteriophages and other vectors usually used in the field of genetic engineering. Vectors suitable for use in the present invention include, but are not limited to the T7-based expression vector for expression in mammalian cells and baculovirus-derived vectors for expression in insect cells. Preferably, the polynucleotide of the invention is operatively linked to the regulatory elements in the recombinant vector of the invention that guarantee the transcription and synthesis of an mRNA in prokryotic and/or eukaryotic cells that can be translated. The nucleotide sequence to be transcribed can be operably linked to a promoter like a T7, metallothionein I or polyhedrin promoter.

In a further embodiment, the present invention relates to host cells transiently or stably containing the polynucleotides or vectors or the invention. A host cell is understood to be an organism that is capable to take up *in vitro* recombinant DNA and, if the case may be, to synthesize the BTNL2 polypeptides encoded by the polynucleotides of the invention. Preferably, these cells are prokaryotic or eukaryotic cells, for example mammalian cells, bacterial cells, insect cells or yeast cells.

The present invention also relates to a method for the recombinant production of a polypeptide-dimer of the invention or a monomer of said dimer, whereby a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., an anti-BTNL2 antibody, or, e.g., can be substantially purified by the one-step method described in Smith and Johnson, Gene 67; 31-40 (1988).

The present invention also relates to a pharmaceutical composition comprising a polypeptide-dimer, polynucleotide or recombinant vector according to the present invention and a pharmaceutically acceptable excipient, diluent or carrier.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease, its localisation and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the disease (e.g. tumor), general health and other drugs being administered concurrently.

The delivery of the polynucleotides of the invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above polynucleotides include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired polynucleotide. Organ-specific or cell-specific liposomes can be used in order to achieve delivery only to the desired tissue. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods).

Preferred recombinant vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

The present invention also relates to the use of the above compounds of the invention (protein-dimers, polynucleotides, vectors etc.) for the preparation of a pharmaceutical composition for treatment or prevention of an inflammatory disease which is mediated by activated T-cells, preferably multiple sclerosis (MS), myocarditis, arthritis, thyroiditis, myasthenia gravis, diabetes, lupus erythematosus, inflammatory bowel disease, sarcoidosis, myasthenia gravis, graft-versus-host disease (GvHD),cancer, lymphoma, celiac disease or psoariasis.

### Brief description of the Figures

### Figure 1: The concept of co-stimulation during T cell activation

An optimal immunological T cell response after MHC-Ag presentation is only obtained if the TCR-mediated first signal is escorted by the binding of B7-1/2 expressed on APC to T cell expressed CD28 **(C).** Missing presence of B7-1/2 is leading to T cell anergy (A), the binding of B7-1/2 to CD28 alone has no biological effect **(B).** Beside the stimulatory role of B7-1/2 in combination with CD28, binding to CTLA-4 inhibits Ag-MHC-induced T cell activation **(D).** A similar inhibitory mechanism is hypothezised for BTNL2 **(E).**

### Figure 2: Nucleotide and amino acid sequence of the extracellular domain of human BTNL2

### Figure 3: Outline of putative BTNL2 dimers

**(A)** A fusion protein comprising two extracellular domains of BTNL2 which are linked via an IgG-Fc molecule. **(B,C)** BTNL2 dimers linked via polypeptides of variable lengths either over their C-termini **(B)** or via a "head-to-tail" orientation **(C).** In addition, the dimers can be tagged (TAG) at various positions. Tags can be e.g. Hisₙ, c-myc, Strep, FLAG, Poly-Arginine, calmodulin-binding peptide (CBP), chitin-binding domain, glutathione S-transferase (GST), maltose-binding protein (MBP) and others.

### Figure 4: Nucleotide and amino acid sequence of the fusion protein BTNL2-Fc.

The protein comprises the extracellular domain of BTNL2, a linker sequence (underlined) and an IgG-Fc molecule.

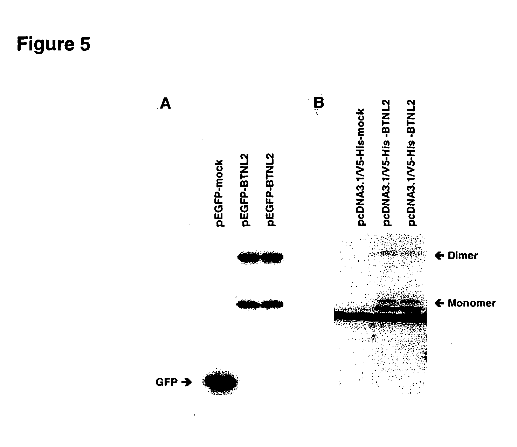

### Figure 5: Expression of BTNL2

Tagged with **(A)** green fluorescence protein (GFP) or V5-His **(B).** Staining of the protein with the respective antibody demonstrates that BTNL2 builds protein dimers which are independent of the tag as demonstrated by the GFP mock control **(A).**

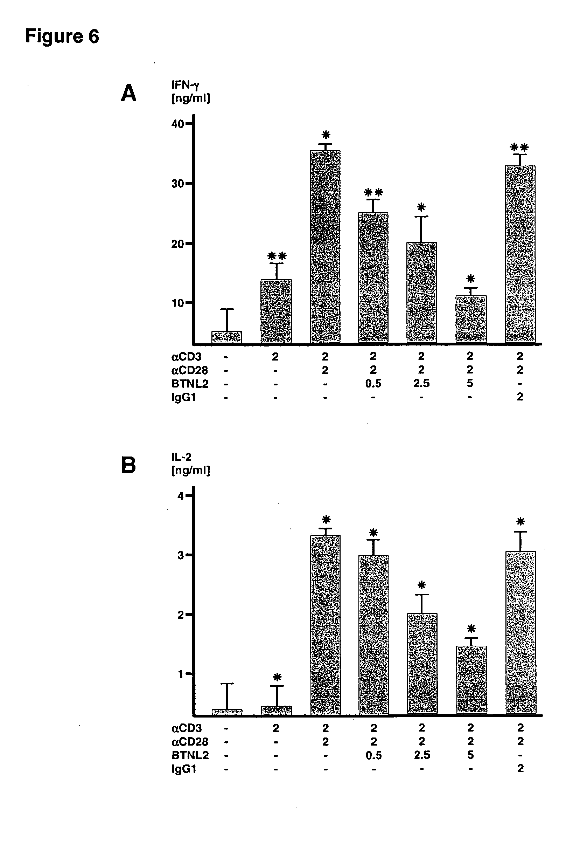

### Figure 6: Secretion of IFN-γ (A) and IL-2 (B) by Jurkat cells after stimulation with anti-CD3 and/or anti-CD28 antibody and increasing amounts of the fusion protein BTNL2-Fc.

Each graph represents the average results of at least 2 independent experiments (comprising 3 identical repeats each).(* p < 0.05; ** p < 0.01).

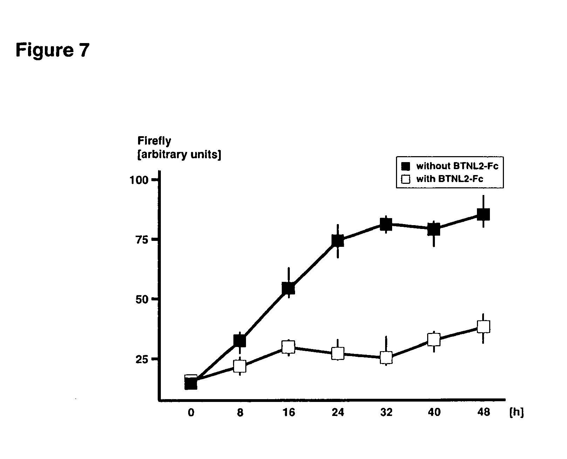

### Figure 7: Inhibition of CD3/CD28-mediated NF-κB activation in Jurkat cells by BTNL2-Fc.

### Example 1: Expression of BTNL2 proteins

### (A) Materials

A DNA fragment encoding the extracellular part of the BTNL2 gene (nucleotide 1 to 1107) was amplified by RT-PCR using the primers BTNL2-s (5'-GTCTCGAGATGGTGGATTTTCCAGGC-3') and BTNL2-a (5'-CTGAATTCTGTTTTCCACAAAAATGTCATCCT-3') (the part binding to the coding sequence is underlined) from monocytic RNA, isolated and purified according to standard techniques. The purified fragment was cloned into various plasmids to generate BTNL2 with different tags: pEGFP-N1 to generate BTNL2-GFP, pcDNA3.1/V5-His-TOPO (Invitrogen, Karlsruhe, Germany) to generate BTNL2-His, pcDNA3.1 containing an IgG-Fc fragment to generate BTNL2-Fc. The resulting constructs were verified by DNA sequencing before further use.

### (B) Cell culture and cell transfection.

CHO cells were purchased from the German Collection of Microorganisms and Cell Cultures (DSMZ, Braunschweig, Germany). All cells were cultured in RPMI + 10% fetal calf serum (FCS). One day before transfection the cells were seeded at a density of 5 x 10⁵ cells/2 ml on 6-well plates. Transfections were performed with Fugene^{™} (Roche, Germany) according to the manufacturer's manual by using 1 µg of the respective BTNL2 expression plasmid described before. 24 to 48 hours after transfection the cells were harvested and further used for protein or RNA isolation. Every single transfection experiment was performed in triplicates and was repeated at least two times.

### (C) BTNL2-Fc forms a homodimer under non-reducing conditions

Supernatants from CHO cells transfected with either a BTNL2-GFP or a BTNL2-His expression plasmid were harvested after 48 h and an aliquot of 25 µl each was resolved on a non-reducing SDS-PAGE. BTNL2-GFP was detected with a GFP-specific monoclonal antibody (BD Bioscience Clontech, Palo Alto,m CA, USA) at a dilution of 1:5000 according to standard techniques (A). BTNL2-His was detected in parallel with an anti-V5-His antibody (Invitrogen) (B). In both experiments the respective vector without insert was used as negative control. Figure 5 demonstrates that BTNL2-GFP and BTNL2-His could be detected as monomers and dimers indicating that BTNL2 spontaneously forms soluble dimers.

### Example 2: Inhibition of CD3/CD28 mediated T cell activation by BTNL2-Fc

### (A) Cells and material

Jurkat cells were obtained from the German Collection of Microorganisms and Cell Cultures (DSMZ, Braunschweig, Germany). The cells were cultured in RPMI 1640 medium supplemented with 10% fetal calf serum, 0.5 µM 2-mercaptoethanol, 2 mM glutamine, 100 U/ml penicillin and 100 µg/ml streptomycin. Anti-CD3 antibody (clone OKT3) and anti-CD28 antibody were purchased from eBioscience (San Diego, USA), (clone 37·51). The NF-κB-Luc reporter plasmid and the Dual-Luciferase Reportergen (DLR) assays were purchased from Promega (Mannheim, Germany). BTNL2-Fc was prepared from supernatants of CHO cells transfected with pcDNA3.1-BTNL2-Fc. The protein was purified by chromatography on a Protein A column (Amersham Bioscience, Buckinghamshire, UK) and subsequent gel filtration (Sephacryl S-300 HR, Amersham).

### (B) Activation of T cells and cytokine detection by ELISA

Microtiter plates were coated with anti-CD3 antibody (2 µg/ml) overnight at 4°C. Jurkat cells (5x10⁴ cells/well) were incubated on these plates in 200 µl of medium for 20 hours at 37°C in the absence or presence of different amounts of BTNL2-Fc. In parallel, an unspecific IgG1 antibody was used instead of BTNL2-Fc as negative control. Subsequently, the cells were incubated with 2 µg/ml of anti-CD28 antibody for another 36 hours. IL-2 and IFN-γ secretion was measured in the supernatants by ELISA according standard techniques. Each experiment was performed in triplicates and was replicated at least two times. The results are depicted in Figure 6.

### (C) Inhibition of NF-κB activation in T cells by BTNL2-Fc

The intracellular signalling molecule NF-KB was recently described to become activated by CD3/CD28 costimulation (Schmitz, et al., Faseb J, 2003, 17:2187). One day before transfection Jurkat cells were seeded at a density of 5 x 10⁵ cells/2 ml on 6-well plates. Transfections were performed with Polyfect^{™} (Qiagen, Hilden, Germany) according to the manufacturer's manual by using 1.8 µg of the NF-κB-Luc reporter plasmid (BD Biosciences) and 0.2 µg of the pRL-TK reference plasmid (Promega, CA, USA). The cells were then transfered to anti-CD3 coated 6-well plates (see (B)) and incubated for 20 hours at 37°C in the presence of 2.5 µg/ml BTNL2-Fc. 2 µg/ml anti-CD28 antibody was added and the cells were incubated for up to 48 hours at 37°C. Parts of the cells were harvested at the indicated time points (Figure 7), lysed with passive lysis buffer (PLB) and luciferase activity was measured according to the manufacturers protocol and normalized to the renilla luciferase results.

In summary, the expression of pro-inflammatory cytokines such as IL-2 and IFN-γ by co-stimulated T-cells can be downregulated by the treatment of the cells with BTNL2-Fc in a dose-dependent manner. This downregulation of cytokine expression is most likely mediated by the specific inhibition of NFκB activity as shown in Figure 6. The results indicate that BTNL2-Fc treatment might have therapeutic potential for the treatment of T cell-mediated diseases by downregulation of T cell activity and pro-inflammatory mediators.

## Claims

1. A polypeptide-dimer comprising at least one soluble BTNL2 molecule.

2. The polypeptide-dimer of claim 1 comprising two soluble BTNL2 molecules.

3. The polypeptide-dimer of claim 2, wherein at least one of the two soluble BTNL2 molecules is the entire extracellular domain of BTNL2.

4. The polypeptide-dimer of any one of claims 1 to 3, wherein the two BTNL2 molecules are linked to each other through a simple covalent bond.

5. The polypeptide-dimer of any one of claims 1 to 3, wherein the two BTNL2 molecules are linked to each other through a flexible peptide linker.

6. The polypeptide-dimer of any one of claims 1 to 5, wherein each of the two BTNL2 molecules is fused to the Fc domain of an IgG protein.

7. The polypeptide-dimer of claim 6 that is expressed as disulfide-linked homodimer.

8. A polynucleotide encoding the polypeptide-dimer of any one of claims 1 to 7 or a monomer of said dimer.

9. An expression vector containing a polynucleotide of claim 8.

10. A host cell containing an expression vector of claim 9.

11. A method of producing a polypeptide-dimer of any one of claims 1 to 7, comprising culturing a host cell of claim 10 and recovering the polypeptide-monomer or -dimer from said host cell or the culture medium.

12. A pharmaceutical composition containing a polypeptide-dimer of any of claims 1 to 7, a polynucleotide of claim 8 or an expression vector of claim 9.

13. Use of a polypeptide-dimer of any of claims 1 to 7, a polynucleotide of claim 8 or an expression vector of claim 9 for the preparation of a pharmaceutical composition for the treatment or prevention of an inflammatory disease which is mediated by activated T-cells.

14. The use of claim 13, wherein said disease is multiple sclerosis (MS), myocarditis, arthritis, thyroiditis, myasthenia gravis, diabetes, lupus erythematosus, inflammatory bowel disease, sarcoidosis, myasthenia gravis, graft-versus-host disease (GvHD), cancer, lymphoma, celiac disease or psoriasis.
